# EUROPÄISCHE PATENTSCHRIFT

(11) **EP 4 106 557 B1**
(45) Veröffentlichungstag und Bekanntmachung des Hinweises auf die Patenterteilung: **11.03.2026**
(21) Anmeldenummer: 21706252.0
(22) Anmeldetag: 17.02.2021
(51) Int. Cl.: A24C 5/34, A24D 3/02, G01N 21/47, G01N 33/00, B65B 19/30

(54) **VERFAHREN UND VORRICHTUNG ZUR PRÜFUNG VON STABFÖRMIGEN PRODUKTEN DER ZIGARETTENINDUSTRIE**
METHOD AND DEVICE FOR EXAMINING ROD-SHAPED PRODUCTS OF THE CIGARETTE INDUSTRY
PROCÉDÉ ET DISPOSITIF D'EXAMEN DE PRODUITS EN FORME DE TIGE DE L'INDUSTRIE DES CIGARETTES

(30) Priorität: 21.02.2020 DE 102020001136
(43) Veröffentlichungstag der Anmeldung: 28.12.2022
(73) Patentinhaber: Focke & Co. (GmbH & Co. KG), 27283 Verden (DE)
(72) Erfinder: CZARNOTTA, Michael, 28279 Bremen (DE); BREITHAUPT, Karl-Heinz, 27313 Dörverden (DE)
(74) Vertreter: Aulich, Martin
(86) Internationale Anmeldenummer: PCT/EP2021/053860
(87) Internationale Veröffentlichungsnummer: WO 2021/165303

(56) Entgegenhaltungen:
- EP-A1- 0 553 699
- DE-A1- 102016 013 719
- DE-A1- 2 411 231
- DE-A1- 2 653 298
- DE-A1- 3 243 204

## Beschreibung

Die vorliegende Erfindung betrifft ein Verfahren zur Prüfung von stabförmigen Produkten der Zigarettenindustrie, insbesondere von Tabaksticks, mit jeweils einer insbesondere zylindrischen Mantelfläche und zwei an gegenüberliegenden Enden angeordneten, insbesondere durch zwei Filterelemente vorzugsweise unterschiedlicher Länge gebildeten, insbesondere visuell identischen Stirnseiten, wobei das stabförmige Produkt zwischen den beiden Stirnseiten, bevorzugt in einem außermittig angeordneten Abschnitt, rauchbares Material aufweist, insbesondere Tabak. Weiter betrifft die Erfindung eine Vorrichtung zur Prüfung derartiger Produkte.

In der Tabak- bzw. Zigarettenindustrie sind diverse Prüfverfahren für Zigaretten oder andere stabförmige Produkte bekannt, die regelmäßig im Rahmen des Verpackungsprozesses solcher Produkte Anwendung finden. In einem solchen Verpackungsprozess werden die stabförmigen Produkte häufig zu separaten Produktgruppen mit ein oder mehreren Produktlagen zusammengefasst, in denen die Zigaretten nebeneinander liegen und in einer bestimmten und gleichen Weise ausgerichtet sind. Herkömmliche Zigaretten werden in einer solchen Produktgruppe in der Regel so ausgerichtet, dass deren durch die Filterenden gebildeten Stirnseiten zusammen eine erste Produktgruppenseite bilden und deren durch die Tabakenden gebildeten Stirnseiten eine gegenüberliegende zweite Produktgruppenseite. Die Produktgruppen werden dann in die jeweilige Verpackung integriert, beispielsweise in eine herkömmliche Zigarettenpackung. In diesem Zusammenhang ist es unter anderem notwendig zu erkennen, ob in einer Produktgruppe einzelne Zigaretten fehlerhafterweise um 180° gedreht angeordnet sind, ob also beispielsweise das Filterende einer Zigarette in der Produktgruppe fehlerhafterweise der Produktgruppenseite zugeordnet ist, die durch die Tabakenden der Zigaretten gebildet ist. Hierfür ist es beispielsweise bekannt, mit einer Kamera ein Bild der Filterenden der Zigaretten einer Produktgruppe aufzunehmen und darin mittels Bilderkennungstechniken ein fälschlich vorhandenes Tabakende zu identifizieren.

Dieses Verfahren versagt allerdings, wenn die beiden Enden der stabförmigen Produkte visuell nicht oder nur unzureichend voneinander unterscheidbar sind. Dies kann zum Beispiel der Fall sein bei neuartigen Produkten der Zigarettenindustrie, wie etwa Tabaksticks ("Heat not Burn"), von denen Ausführungen bekannt sind, bei denen beide Enden durch Filter gebildet werden, deren Stirnseiten visuell identisch sind.

Denkbar wäre in einem solchen Fall, die Mantelflächen der jeweiligen stabförmigen Produkte mit außermittigen Aufdrucken zu versehen, mittels Kameras die Mantelflächen und somit die Aufdrucke zu erfassen und auf diese Weise Fehlorientierungen von einzelnen stabförmigen Produkten in einer Produktgruppe zu erkennen, da in einem solchen Fall der jeweilige Aufdruck an einer falschen bzw. nicht erwarteten Position in dem aufgenommen Bild erscheinen würde. Dieses Verfahren wäre allerdings aufwendig, da sämtliche stabförmigen Produkte mit einem Aufdruck versehen werden müsste. Zudem wäre es bei Produktgruppen mit drei oder mehr Produktlagen nicht mehr fehlerfrei anwendbar, da die mittleren Produktlagen von den äußeren Produktlagen überdeckt werden und somit nicht mit Kameras geprüft werden können.

In der DE 26 53 298 A1 werden nacheinander einzelne Zigaretten geprüft, indem ein Lichtstrahl auf das jeweilige Ende einer Zigarette gerichtet wird und ein Lichtdetektor ein Lichtsignal empfängt, welches eine Tabakmenge oder einen vorhandenen Filter signalisiert. Aus diesem Dokument ist ein Verfahren und eine Vorrichtung mit den Merkmalen im Oberbegriff der vorliegenden Ansprüche 1 und 10 bekannt.

Die DE 24 11 231 A1 zeigt eine Vorrichtung, bei der Zigarettenfilter von Zigaretten geprüft werden, die auf einem Fördermedium einzeln durch eine Prüffläche hindurch gefördert werden, in der der Zigarettenfilter jeweils durch mindestens eine Lichtquelle von mehreren Seiten beleuchtet wird. Ein Photosensor empfängt aus dem Zigarettenfilter austretendes Licht und wandelt dieses in ein Signal für die empfangene Lichtmenge um.

Die DE 10 2016 013719 A1 beschreibt das Prüfen von mehrlagigen Zigarettengruppen mittels einer Bildleiteinrichtung, die es ermöglicht, dass gleichzeitig Bilder oder Teilbilder von mindestens zwei Seiten der Zigarettengruppe zu einem Prüforgan geleitet werden. Dadurch kann eine Bildaufnahme erzeugt werden, die diese mindestens zwei Bilder bzw. Teilbilder umfasst.

Ausgehend hiervon ist es Aufgabe der vorliegenden Erfindung, das eingangs genannte Verfahren und die eingangs genannte Vorrichtung weiterzuentwickeln. Insbesondere derart, dass auch stabförmige Produkte mit optisch nicht oder nur unzureichend unterscheidbaren Enden bzw. Stirnseiten zuverlässig geprüft werden können.

Diese Aufgabe wird gelöst durch ein Verfahren mit den Merkmalen des Anspruchs 1 und einer Vorrichtung mit den Merkmalen des Anspruchs 10.

Dementsprechend wird ein Verfahren zur Prüfung der eingangs genannten stabförmigen Produkte der Zigarettenindustrie zur Verfügung gestellt mit folgenden Maßnahmen:
a) Bestrahlen der Mantelfläche mindestens eines zu prüfenden stabförmigen Produkts mit Prüflicht, derart, dass das Prüflicht durch die Mantelfläche hindurch mindestens teilweise in das stabförmige Produkt eindringt und mindestens teilweise durch eine Stirnseite wieder austritt, insbesondere mit Prüflicht, dessen Strahlen unter einem Winkel von mindestens 30°, bevorzugt von mindestens 60°, besonders bevorzugt von 90° zur Längserstreckung des stabförmigen Produkts auf die Mantelfläche auftreffen,
b) Erfassen des aus der Stirnseite des zu prüfenden stabförmigen Produkts austretenden Prüflichts mit einem der Stirnseite zugeordneten, elektrooptischen Empfänger für das Prüflicht, dessen Hauptblickrichtung auf die Stirnseite gerichtet ist, insbesondere einer Kamera,
c) Auswerten des aus der Stirnseite ausgetretenen, von dem elektrooptischen Empfänger erfassten Prüflichts, insbesondere im Hinblick auf dessen Helligkeit bzw. Lichtintensität.

Es hat sich gezeigt, dass das vorgenannte optische Prüfverfahren eine Identifizierung der Orientierung von stabförmigen Produkten in vielen Fällen auch dann ermöglicht, wenn die Stirnseiten visuell identisch sind, etwa wenn beide Enden Filterelemente aufweisen, wie beispielsweise bei Tabaksticks für elektrische Tabakerhitzer (Heat not Burn). Insbesondere wenn, etwa aufgrund unterschiedlicher Längen der beiden Filterelemente, der Abschnitt rauchbaren Materials außermittig in dem stabförmigen Produkt angeordnet ist, also nicht symmetrisch zur senkrecht zur Längsachse verlaufenden Mittelebene des stabförmigen Produkts angeordnet ist, führt dies zu einer Lichtabsorption (für das Prüflicht) entlang der Längsachse des stabförmigen Produkts, also von der einen Stirnseite zu der anderen Stirnseite, die richtungsabhängig unterschiedlich ist. Diese Richtungsabhängigkeit der Absorption kann dann erfindungsgemäß ausgenutzt werden. Aber auch bei zwei Filterelementen gleicher Länge und mittiger Lage des Abschnitts rauchbaren Materials ist theoretisch eine solche richtungsabhängige Absorption denkbar. Etwa wenn in diesem Abschnitt insbesondere außermittig ein zusätzliches absorbierendes Element enthalten ist, wie etwa eine Aromakapsel oder dergleichen.

Es versteht sich, dass das erfindungsgemäße Verfahren auch bei konventionellen Zigaretten mit einem Tabakende und einem Filterende verwendet werden kann.

Stabförmige Produkte der Zigarettenindustrie können im Rahmen der Anmeldung entsprechend grundsätzlich konventionelle stabförmige Produkte mit rauchbarem Material sein, wie etwa Zigaretten, Zigarillos oder dergleichen, aber auch alternative stabförmige Produkte mit rauchbarem Material, wie etwa Tabaksticks für elektrische Tabakerzeuger (mit oder ohne Aromakapsel) oder dergleichen.

Insofern in einer bevorzugten Ausführungsform der Erfindung ein oder mehrere stabförmige Produkte mit jeweils einem außermittig angeordneten Abschnitt von rauchbarem Material geprüft werden, kann die jeweilige Mantelfläche des jeweiligen stabförmigen Produkts derart mit Prüfstrahlen des Prüflichts bestrahlt werden, dass die Prüfstrahlen nach dem Eindringen in die Mantelfläche auf ihrem Weg zu dem elektrooptischen Empfänger in einer ersten Orientierung des jeweiligen stabförmigen Produkts kein rauchbares Material oder einen kürzeren Abschnitt rauchbaren Materials innerhalb des stabförmigen Produkts durchdringen oder durchdringen würden, während sie - bei unverändertem Prüflicht bzw. bei räumlich unverändertem Strahlweg des auf die Mantelfläche auftreffenden Prüflichts - in einer zweiten, um 180° entgegengesetzten Orientierung des jeweiligen stabförmigen Produkts rauchbares Material bzw. einen längeren Abschnitt rauchbaren Materials innerhalb des stabförmigen Produkts durchdringen oder durchdringen würden. Hieraus ergeben sich dann jeweils abhängig von der Orientierung des stabförmigen Produkts entsprechend aufgrund unterschiedlicher Absorptionen der Prüfstrahlen unterschiedliche Lichtintensitäten an dem Empfänger, woraus die Ist-Orientierung des jeweiligen stabförmigen Produkts ermittelt werden kann.

Bevorzugt kann die Helligkeit bzw. Lichtintensität des erfassten Prüflichts verglichen werden mit einem in einem Speicher hinterlegten Referenzwert oder mit der Helligkeit bzw. Lichtintensität von aus der anderen Stirnseite am gegenüberliegenden Ende desselben stabförmigen Produkts austretendem Prüflicht oder von aus einer Stirnseite eines anderen stabförmigen Produkts austretendem Prüflicht, dessen Mantelfläche derart mit Prüflicht bestrahlt wird, dass das Prüflicht durch die Mantelfläche hindurch mindestens teilweise in das stabförmige Produkt eindringt und mindestens teilweise durch die Stirnseite wieder austritt, und dessen aus der Stirnseite austretendes Prüflicht mit einem oder dem elektrooptischen Empfänger erfasst und ausgewertet wird.

Erfindungsgemäß wird eine gesamte Produktgruppe oder Produktformation aus stabförmigen Produkten geprüft, die mehrere Produktlagen von jeweils in einer Reihe nebeneinander angeordneten stabförmigen Produkten aufweist, deren Stirnseiten an ihren gegenüberliegenden Enden jeweils eine von zwei gegenüberliegenden Produktgruppenseiten bilden.

Dabei kann die Mantelfläche jedes stabförmigen Produkts einer Produktlage der Produktgruppe, vorzugsweise einer oder beider äußeren Produktlagen, mit dem Prüflicht bestrahlt werden, wobei das Prüflicht, das aus den Stirnseiten der stabförmigen Produkte von mindestens einer der beiden gegenüberliegenden Produktgruppenseiten austritt, von einem bzw. dem diesen Stirnseiten zugeordneten elektrooptischen Empfänger empfangen und ausgewertet wird.

Jeder der gegenüberliegenden Stirnseiten oder der stabförmigen Produkte kann jeweils ein insbesondere gesonderter elektrooptischer Empfänger zum Erfassen des jeweils austretenden Prüflichts zugeordnet sein, wobei sowohl das aus der einen als auch das aus der anderen der gegenüberliegenden Stirnseiten ausgetretene, von dem jeweils zugeordneten elektrooptischen Empfänger erfasste Prüflicht ausgewertet wird.

Was den (jeweiligen) zugeordneten elektrooptischen Empfänger betrifft, so kann dessen Hauptblickrichtung senkrecht zu den Stirnseite(n) der stabförmigen Produkte verlaufen.

Der oder der jeweilige zugeordnete elektrooptische Empfänger kann vorzugsweise als Kamera ausgebildet sein, insbesondere als CCD- oder CMOS-Kamera, wobei die oder die jeweilige Kamera ein insbesondere gemeinsames Ist-Bild der einen oder mehreren Stirnseite(n) aufnimmt, und wobei das oder das jeweilige Ist-Bild dann ausgewertet wird.

Dabei kann im Rahmen der Auswertung des Ist-Bildes die Helligkeit oder Lichtintensität einer darin abgebildeten Stirnseite eines stabförmigen Produkts verglichen werden mit einem hinterlegten Referenzwert. Alternativ kann im Rahmen der Auswertung des Ist-Bildes die Helligkeit oder Lichtintensität einer darin abgebildeten Stirnseite verglichen werden mit der jeweiligen Helligkeit bzw. Lichtintensität mindestens einer anderen darin abgebildeten Stirnseite eines stabförmigen Produkts derselben Produktlage oder einer anderen Produktlage.

Jeder der Stirnseiten an den beiden Enden des stabförmigen Produkts oder jedes stabförmigen Produkts einer oder der Produktgruppe kann jeweils ein insbesondere gesonderter elektrooptischer, als Kamera ausgebildeter Empfänger zum Erfassen des jeweils austretenden Prüflichts zugeordnet sein. Insbesondere derart, dass die Hauptblickrichtung der jeweiligen Kamera senkrecht zu der jeweils zugeordneten Stirnseite ausgerichtet ist, dass jede Kamera jeweils ein Ist-Bild derjenigen Stirnseite aufnimmt, der sie zugeordnet ist, und dass diese Ist-Bilder ausgewertet werden, indem die Helligkeit oder Lichtintensität der in dem einen Ist-Bild abgebildeten Stirnseite des einen Endes des stabförmigen Produkts verglichen wird mit der Helligkeit bzw. Lichtintensität der in dem anderen Ist-Bild abgebildeten Stirnseite des anderen Endes des stabförmigen Produkts.

Vorzugsweise kann erfindungsgemäß ein Fehlersignal erzeugt werden für den Fall, dass im Rahmen des Vergleichs der Helligkeit der einen Stirnseite mit der jeweiligen Helligkeit bzw. Lichtintensität der anderen Stirnseite ein Unterschied zwischen den Helligkeits- bzw. Lichtintensitätswerten festgestellt wird, der über ein vorbestimmtes Maß hinaus geht.

Die oder jede Mantelfläche der jeweiligen stabförmigen Produkte kann aus zwei verschiedenen Richtungen mit Prüflicht beaufschlagt werden, insbesondere aus um 180° entgegengesetzten Richtungen.

Weiter kann bei Prüfung einer Produktgruppe mit mehreren Produktlagen vorgesehen sein, die Mantelflächen der stabförmigen Produkte zweier äußerer Produktlagen der Produktgruppe jeweils (von unterschiedlichen Seiten aus) mit Prüflicht zu beaufschlagen, wobei das Prüflicht, mit dem die eine äußere Produktlage bestrahlt wird, aus einer anderen Richtung stammt als das Prüflicht, mit dem die andere äußere Produktlage bestrahlt wird, insbesondere aus um 180° entgegengesetzten Richtungen.

Für den Fall, dass ein oder mehrere stabförmige Produkte mit jeweils einem außermittig angeordneten Abschnitt von rauchbarem Material geprüft werden, kann vorgesehen sein, die jeweilige Mantelfläche des jeweiligen stabförmigen Produkts derart mit Prüfstrahlen des Prüflichts zu bestrahlen, dass die Prüfstrahlen nach dem Eindringen in die Mantelfläche auf ihrem Weg zu dem elektrooptischen Empfänger in einer ersten Orientierung des jeweiligen stabförmigen Produkts kein rauchbares Material oder einen kürzeren Abschnitt rauchbaren Materials innerhalb des stabförmigen Produkts durchdringen oder durchdringen würden, während sie in einer zweiten, um 180° entgegengesetzten Orientierung des jeweiligen stabförmigen Produkts rauchbares Material bzw. einen längeren Abschnitt rauchbaren Materials innerhalb des stabförmigen Produkts durchdringen oder durchdringen würden.

Eine geeignete Vorrichtung zur Prüfung der stabförmigen Produkte, insbesondere zur Durchführung des obigen Verfahrens, weist mindestens eine Beleuchtungseinrichtung auf, von der Prüflicht ausgeht, das auf die Mantelfläche mindestens eines zu prüfenden stabförmigen Produkts gerichtet ist, bevorzugt derart, dass dessen Strahlen unter einem Winkel von mindestens 30°, bevorzugt von mindestens 60°, besonders bevorzugt von 90° zur Längserstreckung des stabförmigen Produkts auf die Mantelfläche auftreffen, wobei das Prüflicht durch die Mantelfläche hindurch mindestens teilweise in das stabförmige Produkt eindringt und mindestens teilweise durch die Stirnseite wieder austritt, mindestens einen elektrooptischen Empfänger, der derart angeordnet ist, dass dessen Hauptblickrichtung zur Erfassung desselben auf die Stirnseite des stabförmigen Produkts gerichtet ist, sodass das aus der Stirnseite des stabförmigen Produkts austretende Prüflicht erfasst wird, und eine Auswerteeinrichtung, mit der das von dem elektrooptischen Empfänger empfangene Prüflicht auswertbar ist, insbesondere im Hinblick auf dessen Helligkeit bzw. Lichtintensität, bevorzugt derart, wie es oben beschrieben ist.

Erfindungsgemäß ist das Prüflicht auf eine Produktgruppe aus stabförmigen Produkten gerichtet, die mehrere Produktlagen von jeweils in einer Reihe nebeneinander angeordneten stabförmigen Produkten aufweist, deren Stirnseiten an ihren gegenüberliegenden Enden jeweils eine von zwei gegenüberliegenden Produktgruppenseiten bilden, wobei die Beleuchtungseinrichtung dann derart ausgebildet ist, dass die Mantelflächen der stabförmigen Produkte mindestens einer Produktlage, insbesondere einer oder beiden äußeren Produktlagen, mit dem Prüflicht beaufschlagbar sind, während sich die Produktgruppe in einer Aufnahme eines Förderers befindet, insbesondere in der Tasche eines drehbaren Revolvers.

Weiter vorzugsweise kann eine Beleuchtungseinrichtung vorgesehen sein, die die Mantelflächen einer ersten äußeren Produktlage der Produktgruppe von einer ersten Seite aus bzw. aus einer ersten Richtung mit Prüflicht beaufschlagt und die Mantelflächen einer zweiten äußeren Produktlage der Produktgruppe von einer zweiten, anderen Seite aus bzw. aus einer zweiten, unterschiedlichen Richtung.

Zu diesem Zweck kann die Beleuchtungseinrichtung mindestens zwei Lichtquellen aufweisen, wobei Prüflicht der einen Lichtquelle die eine äußere Produktlage bestrahlt und Prüflicht der anderen Lichtquelle die andere äußere Produktlage.

Die Beleuchtungseinrichtung kann dabei über eine Lichtleiteinrichtung mit ein oder mehreren Lichtleitorganen verfügen, wie etwa Spiegeln oder Lichtwellenleitern, die das von der einen Lichtquelle der Beleuchtungseinrichtung ausgehende Prüflicht auf die Mantelflächen der stabförmigen Produkte der einen äußeren Produktlage der Produktgruppe leiten und das von der anderen Lichtquelle ausgehende Prüflicht auf die Mantelflächen der stabförmigen Produkte der anderen äußeren Produktlage der Produktgruppe.

Denkbar ist aber auch, dass das Prüflicht ein und derselben Lichtquelle der Beleuchtungseinrichtung von der Lichtleiteinrichtung sowohl zu der einen als auch zu der anderen äußeren Produktlage geleitet wird und diese (von unterschiedlichen Seiten aus bzw. aus unterschiedlichen Richtungen) beaufschlagt.

Die oben genannten zwei Lichtquellen der Beleuchtungseinrichtung oder mindestens zwei Lichtleitorgane der Lichtleiteinrichtung der Beleuchtungseinrichtung können benachbart zu unterschiedlichen Seiten der Produktgruppe angeordnet sein, insbesondere derart, dass Prüflicht aus unterschiedlichen, vorzugsweise aus um 180° entgegengesetzten Richtungen die Mantelflächen der ersten bzw. der zweiten Produktlage beaufschlagt.

Jeder der gegenüberliegenden Stirnseiten der stabförmigen Produkte kann im Übrigen jeweils ein insbesondere gesonderter elektrooptischer Empfänger zum Erfassen des jeweils austretenden Prüflichts zugeordnet sein, insbesondere derart, dass die Hauptblickrichtung des jeweiligen elektrooptischen Empfängers senkrecht zu der den jeweils zugeordneten Stirnseiten ausgerichtet ist.

Jeder Produktgruppenseite, die von den Stirnseiten der gegenüberliegenden Enden der stabförmigen Produkte gebildet wird, kann auch ein gesonderter elektrooptischer Empfänger zum Erfassen des jeweils aus sämtlichen Stirnseiten der jeweiligen Produktgruppenseite austretenden Prüflichts zugeordnet sein, insbesondere derart, dass die Hauptblickrichtung des jeweiligen elektrooptischen Empfängers senkrecht zu den Stirnseiten der jeweiligen Produktgruppenseite ausgerichtet ist.

Weitere Merkmale der vorliegenden Erfindung ergeben sich aus den beigefügten Patentansprüchen, der nachfolgenden Beschreibung bevorzugter Ausführungsbeispiele sowie den beigefügten Zeichnungen.

Darin zeigt:
- Fig. 1: eine erste Ausführungsform einer erfindungsgemäßen Prüfvorrichtung zur Prüfung von Produktgruppen von stabförmigen Tabakprodukten, positioniert im Bereich eines Revolvers zur Förderung der Produktgruppen, perspektivisch,
- Fig. 2: einen Schnitt entlang der Schnittebene II-II in Fig. 1,
- Fig. 3: Bestandteile der Prüfvorrichtung aus Fig. 1 in isolierter Darstellung, insbesondere eine Kamera, die eine von einer Beleuchtungseinrichtung beleuchtete Produktgruppe erfasst, perspektiv,
- Fig. 4a: eine Darstellung analog zu Fig. 3, wobei zwei in derselben Lage der Produktgruppe angeordnete stabförmige Produkte in falscher Orientierung (um 180° gedreht) in der Produktgruppe angeordnet sind,
- Fig. 4b: eine Darstellung analog zu Fig. 3, wobei in beiden Lagen der Produktgruppe stabförmige Produkte in falscher Orientierung angeordnet sind,
- Fig. 5: ein erstes alternatives Ausführungsbeispiel einer erfindungsgemäßen Prüfvorrichtung in Schnittdarstellung,
- Fig. 6: ein zweites alternatives Ausführungsbeispiel einer erfindungsgemäßen Prüfvorrichtung in Schnittdarstellung,
- Fig. 7: ein drittes alternatives Ausführungsbeispiel einer erfindungsgemäßen Prüfvorrichtung in einer Darstellung analog zu Fig. 3.

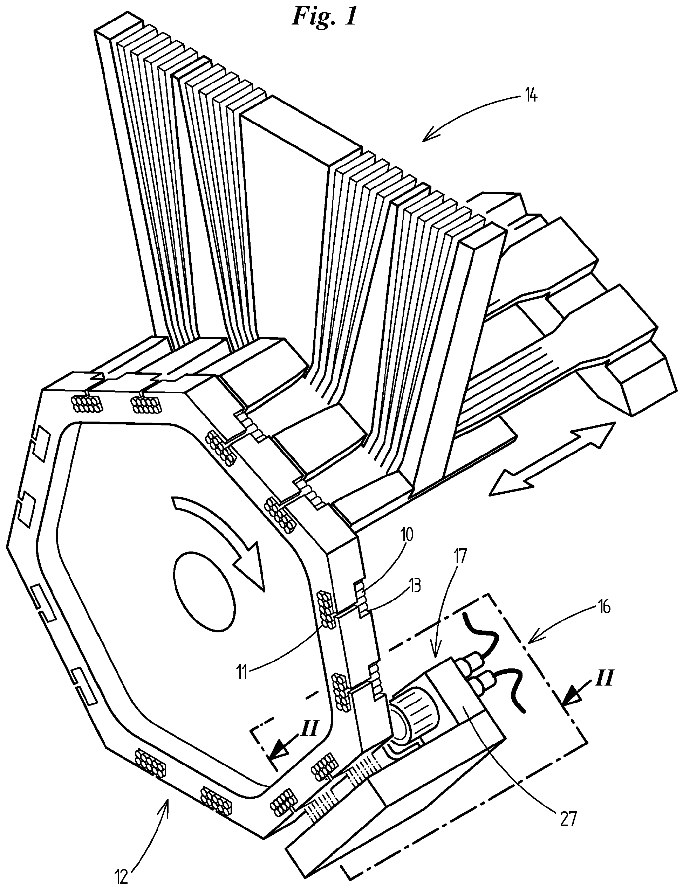

Das erfindungsgemäße Prüfverfahren und die erfindungsgemäße Prüfvorrichtung wird vorliegend im Zusammenhang mit der Prüfung von Produktgruppen 10 stabförmiger Produkte 11, nämlich vorliegend Tabaksticks, erläutert, die in den Ausführungsbeispielen der Fig. 1-7 jeweils in Aufnahmen 13 eines drehend bewegten Förderers 12, nämlich vorliegend eines Revolvers, gefördert werden.

Die stabförmigen Produkte 11 verfügen dabei vorliegen über eine insbesondere zylindrische Mantelfläche 22 sowie an beiden Enden über sich quer zur Längserstreckung des jeweiligen stabförmigen Produkts 11 erstreckende Stirnseiten 23 und 24.

Die Stirnseiten 23, 24 sind gebildet durch entsprechende Stirnseiten von an sich bekannten Filterelementen 20 bzw. 21.

Die Stirnseiten 23, 24 bzw. die Stirnseiten der Filterelemente 20, 21 begrenzen entsprechend die Mantelfläche 22 des jeweiligen stabförmigen Produkts 11 axial.

Zwischen den beiden Filterelementen 20, 21 bzw. zwischen den entsprechenden Stirnseiten 23, 24 des jeweiligen stabförmigen Produkts 11 befindet sich ein Abschnitt 25 mit rauchbarem Material 26. Bei dem rauchbaren Material 26 kann es sich beispielsweise um klassischen Tabak handeln oder um rauchbare Liquids oder dergleichen.

Dieser Abschnitt 25 mit rauchbarem Material 26 ist bezogen auf die senkrecht zur Längsachse des stabförmigen Produkts 11 verlaufende Mittelebene des jeweiligen stabförmigen Produkts 11 vorliegend außermittig bzw. entsprechend nicht symmetrisch zu dieser Mittelebene angeordnet.

Im vorliegenden Fall ist der Abschnitt 25 jenseits der Mittelebene des stabförmigen Produkts 11 vollständig in einer Hälfte desselben angeordnet, insbesondere benachbart zu dem Filterelement 21.

Dabei weisen die Filterelemente 20, 21, zwischen denen der Abschnitt 25 angeordnet ist, unterschiedliche Längen auf.

Zwischen den Filterelementen 20, 21 können zudem noch weitere Elemente angeordnet sein, vorliegend beispielhaft als Füllstück 19 dargestellt.

Die Produktgruppen 10 aus stabförmigen Produkten 11 weisen mehrere Produktlagen 18 aus jeweils in einer Produktlage 18 nebeneinander angeordneten stabförmigen Produkten 11 auf. In den Ausführungsbeispielen der Fig. 1-6 sind dies jeweils zwei Produktlagen 18 mit jeweils fünf stabförmigen Produkten 11. Dies kann aber auch anders sein.

Jede Produktgruppe 10 weist durch die stabförmigen Produkte 11 gebildete Produktgruppenseiten auf:
Eine erste (vordere) Produktgruppenseite 28 wird durch die in einer gemeinsamen Ebene angeordneten Stirnseiten 23 der stabförmigen Produkte 11 gebildet, eine zweite, gegenüberliegende, zu der ersten Produktgruppenseite 28 parallele Produktgruppenseite 29 wird durch die Stirnseiten 24 an den jeweiligen anderen Enden der stabförmigen Produkte 11 gebildet und sind ebenfalls in einer gemeinsamen Ebene angeordnet.

Eine dritte (untere) sowie eine vierte (obere) Produktgruppenseite 35 bzw. 36 werden durch die Mantelflächen 22 der stabförmigen Produkte 11 gebildet bzw. definiert.

Bei korrekter Orientierung der stabförmigen Produkte 11 in der entsprechenden Produktgruppe 10 sind diese so in der Produktgruppe 10 angeordnet bzw. ausgerichtet, dass die jeweiligen gleichartigen Filterelemente 20 bzw. 21 jeweils derselben Produktgruppenseite 28 bzw. 29 zugeordnet sind.

Der oben erwähnte Förderer 12 ist Teil einer nicht dargestellten, im Stand der Technik bekannten Verpackungsmaschine zur Herstellung von Packungen für Produktgruppen 10 stabförmiger Produkte 11 jeweils als Packungsinhalt. Bei solchen Packungen kann es sich beispielsweise um Hing-Lid-Packungen, aber auch um jegliche andere Arten von Packungen handeln, in die Produktgruppen stabförmiger Produkte 11 der Zigaretten- bzw. Tabakindustrie verpackt werden können.

Vorliegend werden dem Förderer 12 die stabförmigen Produkte 11 aus einem Magazin 14 unter Bildung der Produktgruppen 10 zugefördert, und zwar mittels eines Einstößers.

Während sich die Produktgruppen 10 in den Aufnahmen 13 des Förderers 12 befinden, werden sie von einer Prüfeinrichtung 16 in später noch näher beschriebener Weise geprüft.

Es versteht sich, dass die dargestellte Prüfeinrichtung 16 auch in verschiedenen anderen Bereichen der Verpackungsmaschine angeordnet sein könnte bzw. die Prüfung auch an anderen Stellen des Verpackungsprozesses erfolgen könnte.

Theoretisch denkbar ist im Übrigen auch eine Einzelprüfung von stabförmigen Produkten 11, ohne dass diese während der Prüfung innerhalb einer Produktgruppe 10 angeordnet sein müssen.

Nach der Prüfung werden die Produktgruppen 10 zur weiteren Handhabung zu weiteren Aggregaten der Verpackungsmaschine gefördert, die die an sich bekannten weiteren Maßnahmen durchführen zur Verpackung der stabförmigen Produkte 11 bzw. der Produktgruppen 10 in die entsprechende Packungen, wie etwa Hinge-Lid-Packungen oder dergleichen.

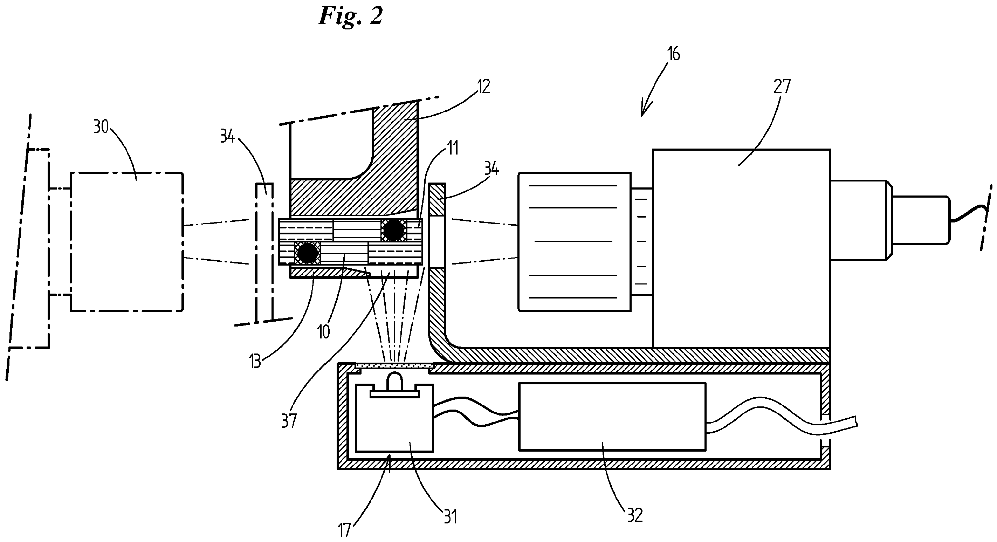

Im vorliegenden Fall wird der Förderer 13 - nachdem die jeweiligen Produktgruppen 10 im Bereich des Magazins 14 in die jeweilige Aufnahme 13 eingeschoben wurden - weiterrotiert, sodass vorliegend zwei als nächstes zu prüfende Produktgruppen 10 jeweils in eine Prüfstellung gefördert werden, vgl. Fig. 2, in der sie von der Prüfeinrichtung 16 geprüft werden.

Zur gruppenweisen Prüfung der zu prüfenden, in der jeweiligen Prüfstellung befindlichen Produktgruppen 10 weist die Prüfeinrichtung 16 zwei Beleuchtungseinrichtungen 17 auf, nämlich für jede zu prüfende Produktgruppe 10 eine, die jeweils eine zugeordnete, in der jeweiligen Prüfposition befindliche Produktgruppe 10 mit Prüflicht beaufschlagen.

Die Beleuchtungseinrichtungen 17 verfügen dabei vorliegend jeweils über geeignete Lichtquellen 31, insbesondere LEDs. Diese werden jeweils von einer Versorgungseinheit 32 mit elektrischem Strom versorgt.

Die Lichtquellen 31 der Beleuchtungseinrichtungen 17 in der Fig. 2 sind jeweils derart benachbart zu den in der jeweiligen Prüfposition befindlichen Aufnahmen 13 des Förderers 12 angeordnet, dass die Prüfstrahlen des Prüflichts winklig zur Längsachse bzw. Längserstreckung der stabförmigen Produkte 11, also nicht parallel zu dieser, auf die Mantelflächen 22 der stabförmigen Produkte 11 der äußeren (radial außen angeordneten) Produktlage 18 der Produktgruppe 10 auftrifft, vorliegend im Wesentlichen senkrecht bzw. in einem Winkel von 90° zu der Längserstreckung der stabförmigen Produkte 11.

Die Lichtquellen 31 der Beleuchtungseinrichtungen 17 sind zu diesem Zweck jeweils in radialem Abstand zu dem Umfang des Förderers 12 positioniert und eine jeweilige radiale Wand der jeweiligen Aufnahme 13 mit einer (radialen) Freimachung 37 versehen, sodass die Mantelflächen 22 der stabförmigen Produkte 11 der äußeren Produktlage 18 durch die Freimachung 37 hindurch mit dem Prüflicht bestrahlt werden können.

Die Prüfvorrichtung 16 weist neben den Beleuchtungseinrichtungen 17 vorliegend zwei elektrooptische Empfänger 27 auf, bevorzugt Kameras. Die elektrooptischen Empfänger 27 sind jeweils derart ausgebildet und ausgerichtet, dass deren Hauptblickrichtungen jeweils auf die Stirnseiten 23 der entsprechenden, durch diese gebildeten Produktgruppenseiten 28 der jeweiligen zu prüfenden Produktgruppen 10 gerichtet ist, nämlich vorliegend senkrecht zu diesen.

Alternativ oder zusätzlich können im Übrigen noch ggf. weitere elektrooptische Empfänger 30 auf der entgegengesetzten Seite des Förderers 12 bzw. der entgegengesetzten Produktgruppenseite 29 angeordnet sein, deren Hauptblickrichtungen jeweils entsprechend auf die Stirnseiten 24 der gegenüberliegenden Produktgruppenseite 29 gerichtet werden würden, vgl. Fig. 2.

Abweichend von der beispielsweise in Fig. 2 gezeigten Darstellung könnten die Beleuchtungseinrichtungen 17 auch so ausgebildet oder angeordnet sein, dass deren Prüflichtstrahlen nicht senkrecht auf die Mantelflächen 22 der stabförmigen Produkte 11 treffen, also nicht unter einem Winkel von 90° relativ zu den Mantelflächen bzw. zur Längsachse der stabförmigen Produkte 11, sondern unter einem anderen, kleineren Winkel, also schräg zu der Längsachse.

Im Prüfprozess wird das Prüflicht der Beleuchtungseinrichtungen 17 in der beschriebenen Weise auf die dritte (untere) Produktgruppenseite 35 geleitet, nämlich auf die Mantelflächen 22 der stabförmigen Produkte 11 der äußeren Produktlage 18.

Dabei werden die stabförmigen Produkte 11 vorliegend vornehmlich im Bereich der (längeren) Filterelemente 20 mit dem Prüflicht bestrahlt, dies muss aber nicht so sein.

Das Prüflicht dringt in die stabförmigen Produkte 11 ein, durchstrahlt diese, wird zum Teil im Inneren abgelenkt und tritt dann zum Teil über die Stirnseiten 23 wieder aus. Dieser aus den Stirnseiten 23 austretende Teil wird durch den jeweiligen elektrooptischen Empfänger 27 erfasst, der ein entsprechendes Bild aufnimmt, indem die Stirnseiten 23 enthalten sind.

Ein anderer Teil des Prüflichts gelangt zu der weiteren bzw. zweiten (radial weiter innen angeordneten) Produktlage 18, tritt teilweise durch die Stirnseiten 23 der stabförmigen Produkte 11 wieder aus und wird ebenfalls von dem jeweiligen bzw. demselben elektrooptischen Empfänger 27 erfasst. Vorzugsweise sind die Stirnseiten 23 der stabförmigen Produkte 11 dieser weiteren, inneren Produktlage 18 in derselben Bildaufnahme enthalten wie die Stirnseiten 23 der ersten, äußeren Produktlage 18.

Zur Verbesserung der Erfassung der Stirnseiten 23 bzw. des aus diesen austretenden Prüflichts ist im Übrigen axial benachbart zu der jeweiligen, in der Prüfposition befindlichen Aufnahme 13 des Förderers 12 im Übrigen noch eine Blende 34 angeordnet, durch die nicht von den Stirnseiten 23 stammendes Streulicht abgefangen wird.

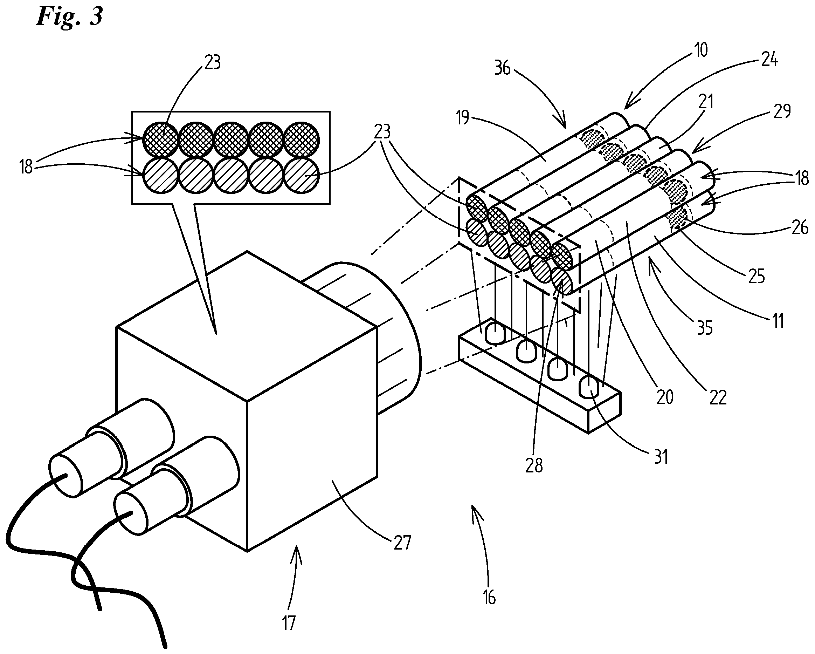

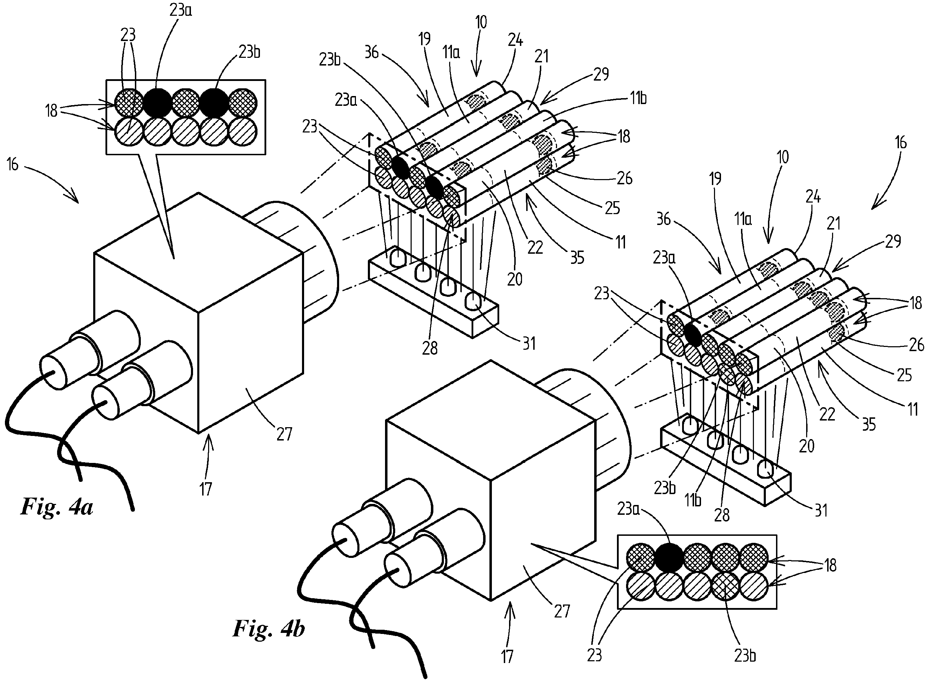

Die Detektion von in nicht korrekter Orientierung in der jeweiligen Produktgruppe 10 angeordneten stabförmigen Produkten 11 wird zunächst beispielhaft anhand der Fig. 3, 4a und 4b erläutert.

In Fig. 3 ist eine Produktgruppe 10 zu erkennen, die ausschließlich korrekt orientierte stabförmige Produkte 11 aufweist. Dabei sind die stabförmigen Produkte 11 der Produktgruppe 10 so angeordnet und orientiert, dass sämtliche (längeren) Filterelemente 20 der Produktgruppenseite 28 zugewandt sind und die (kürzeren) Filterelemente 21 der gegenüberliegenden Produktgruppenseite 29.

Der jeweilige elektrooptische Empfänger 27 nimmt ein (elektronisches) Bild der Produktgruppenseite 28 bzw. sämtlicher Stirnseiten 23 auf. Eine nicht dargestellte Auswerteeinrichtung, beispielsweise ein entsprechender Computer oder eine geeignet ausgerüstete Steuereinrichtung, wertet dann das Bild aus, indem es beispielsweise die Helligkeit bzw. Lichtintensität der jeweiligen Stirnseiten 23 der stabförmigen Produkte 11 einer Produktlage 18 in dem Bild ermittelt und entweder mit hinterlegten Referenzwerten vergleicht oder jeweils mit den ermittelten Helligkeits- bzw. Lichtintensitätswerten ein oder mehrerer anderer Stirnseiten 23 von stabförmigen Produkten 11 derselben Produktlage 18.

Im Rahmen dieses Vergleichs wird dann erkannt, dass sämtliche Stirnseiten 23 in Fig. 3 derselben Produktlage 18 jeweils die gleichen oder zumindest sehr ähnliche Helligkeitswerte aufweisen, was ein Indikator dafür ist, dass alles stabförmigen Produkte 11 in derselben Produktlage 18 in gleicher, korrekter Weise orientiert sind.

Da nur eine bzw. nur die (untere) Produktgruppenseite 35 der Produktgruppe 10 von der jeweiligen Beleuchtungseinrichtung 17 bestrahlt wird, ist im Übrigen auf dem Bild der Stirnseiten 23 zu erkennen, dass die Stirnseiten 23 der äußeren Produktlage 18 heller erscheinen als die Stirnseiten 23 der inneren Produktlage 18, da das Prüflicht auf seinem Weg zu der inneren Produktlage 18 teilweise absorbiert wird.

In Fig. 4a ist eine Situation dargestellt, in der in der äußeren Produktlage 18 zwei falsch orientierte stabförmige Produkte 11a und 11b enthalten sind.

In diesem Fall ergibt die Auswertung des entsprechenden Bildes eine geringere Lichtintensität der zu diesen gehörenden Stirnseiten 23a bzw. 23b. Denn aufgrund der um 180° gedrehten Orientierung der stabförmigen Produkte 11a und 11b trifft das Prüflicht, nachdem es in die stabförmigen Produkte 11a und 11b eingetreten ist, teilweise auf den jeweiligen Abschnitt 25 mit dem rauchbaren Material, wo es stärker absorbiert wird als im Bereich der Filterelemente 20 bzw. 21. Denn Filterelemente sind in der Regel sehr hell ausgebildet und weisen geringere Absorptionswerte auf als rauchbares Material wie Tabak.

Diese geringere Lichtintensität der Stirnseiten 23a bzw. 23b, entweder relativ zu Referenzwerten oder im Vergleich zu ein oder mehreren der anderen Stirnseiten 23 derselben Produktlage 18, ist ein Indikator dafür, dass die beiden stabförmigen Produkte 11a und 11b falsch herum orientiert sind.

Entsprechend kann ein Fehlersignal erzeugt werden und die entsprechende Produktgruppe 10 beispielsweise im Packungsprozess ausgesondert werden.

In ähnlicher Weise kann anhand des entsprechenden aufgenommenen Bildes in dem Beispiel der Fig. 4b erkannt werden, dass in beiden Produktlagen 18 jeweils ein falsch orientiertes stabförmiges Produkt 11a bzw. 11b in der Produktgruppe 11 angeordnet ist.

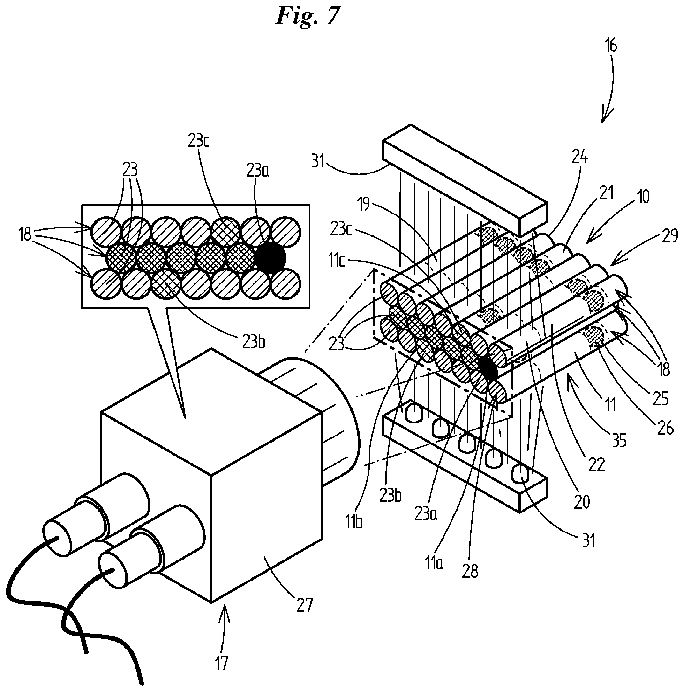

In Fig. 7 ist eine Variante gezeigt, in der die Produktgruppe 10 nicht aus zwei, sondern aus drei Produktlagen 18 gebildet ist. In diesem Fall bietet es sich an, auch die (obere) Produktgruppenseite 36 direkt mit Prüflicht der jeweiligen Beleuchtungseinrichtung 17 zu bestrahlen, da ansonsten in die obere Produktlage 18 ggf. zu wenig Prüflicht eindringen bzw. bei dieser ankommen würde, da das Prüflicht zunächst die in der Fig. 7 untere und mittlere Produktlage 18 durchdringen muss.

Entsprechend sind weitere Lichtquellen 31 der Beleuchtungseinrichtung 17 unmittelbar benachbart zu der (oberen) Produktgruppenseite 36 angeordnet, die die obere Produktlage 18 direkt mit Prüflicht beaufschlagen.

In der in Fig. 7 gezeigten Variante sind - wie an den Stirnseiten 23a, 23b und 23c zu erkennen ist - drei stabförmige Produkte 11a, 11b und 11c falsch orientiert.

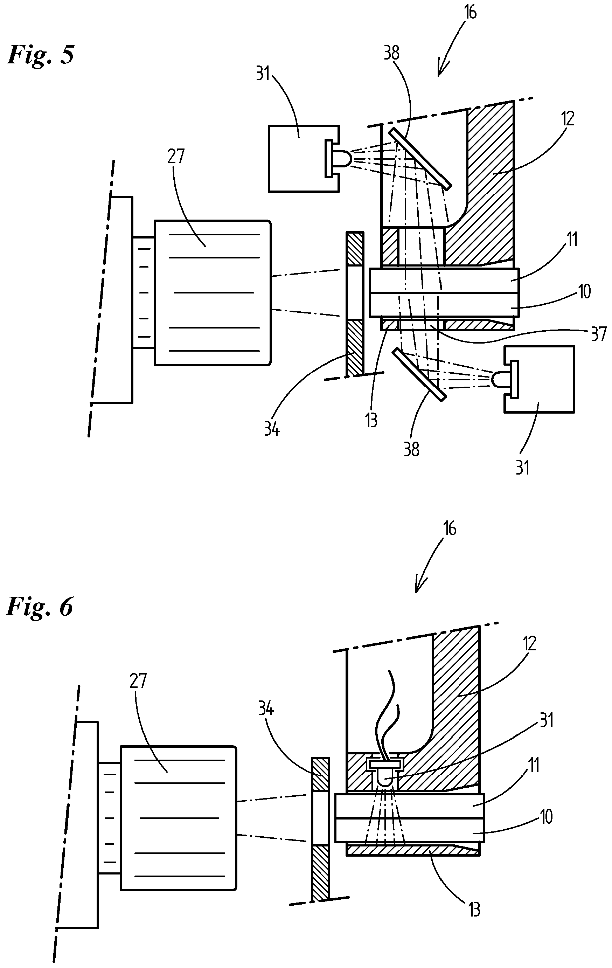

In Fig. 5 ist gezeigt, dass das Prüflicht der jeweiligen Beleuchtungseinrichtung 17, hier ausgehend von mehreren, an unterschiedlichen Seiten der Produktgruppe 10 angeordneten Lichtquellen 31, auch über Lichtleitorgane 38, vorliegend Spiegel (es können aber beispielsweise auch Lichtwellenleiter verwendet werden), einer Lichtleiteinrichtung auf die Produktgruppe 10 geleitet werden kann. Die Lichtleitorgane 38 können dann in geeigneter Weise benachbart zu dem Förderer 12 angeordnet sein, sodass das Prüflicht in der oben beschriebenen Weise auf die Mantelflächen 22 der jeweiligen stabförmigen Produkte 10 der entsprechenden Produktlage 18 auftrifft.

In Fig. 6 ist gezeigt, dass die eine oder die mehreren Lichtquellen 31 auch unmittelbar in den Förderer 12 integriert werden können, sodass die Produktgruppe 10 auch direkt mit dem Prüflicht (ohne Lichtleiteinrichtung) beaufschlagt werden kann.

Was im allgemeinen das Prüflicht betrifft, das mit der oder den Beleuchtungseinrichtungen 17 erzeugt wird, so ist denkbar, dass dieses ggf. die einzelnen stabförmigen Produkte 11 jeweils auch punktuell beaufschlagt, beispielsweise mittels einzelner Laserstrahlen.

**Bezugszeichenliste**

| | | | |
|---|---|---|---|
| 10 | Produktgruppe | 38 | Lichtleitorgan |
| 11 | stabförmiges Produkt | | |
| 11a | stabförmiges Produkt | | |
| 11b | stabförmiges Produkt | | |
| 11c | stabförmiges Produkt | | |
| 12 | Förderer | | |
| 13 | Aufnahme | | |
| 14 | Magazin | | |
| 16 | Prüfeinrichtung | | |
| 17 | Beleuchtungseinrichtung | | |
| 18 | Produktlage | | |
| 19 | Füllstück | | |
| 20 | Filterelement | | |
| 21 | Filterelement | | |
| 22 | Mantelfläche | | |
| 23 | Stirnseite | | |
| 23a | Stirnseite | | |
| 23b | Stirnseite | | |
| 23c | Stirnseite | | |
| 24 | Stirnseite | | |
| 25 | Abschnitt | | |
| 26 | rauchbares Material | | |
| 27 | elektrooptischer Empfänger | | |
| 28 | vordere Produktgruppenseite | | |
| 29 | hintere Produktgruppenseite | | |
| 30 | elektrooptischer Empfänger | | |
| 31 | Lichtquelle | | |
| 32 | Versorgungseinheit | | |
| 34 | Blende | | |
| 35 | untere Produktgruppenseite | | |
| 36 | obere Produktgruppenseite | | |
| 37 | Freimachung | | |

## Patentansprüche

1. Verfahren zur Prüfung von stabförmigen Produkten (11) der Zigarettenindustrie, insbesondere von Tabaksticks, mit jeweils einer insbesondere zylindrischen Mantelfläche (22) und zwei an gegenüberliegenden Enden angeordneten, insbesondere durch zwei Filterelemente vorzugsweise unterschiedlicher Länge gebildeten, insbesondere visuell identischen Stirnseiten (23, 24), wobei das stabförmige Produkt (11) zwischen den beiden Stirnseiten (23, 24), bevorzugt in einem außermittig angeordneten Abschnitt, rauchbares Material aufweist, insbesondere Tabak, mit folgenden Maßnahmen:
a) Bestrahlen der Mantelfläche (22) mindestens eines zu prüfenden stabförmigen Produkts (11) mit Prüflicht, derart, dass das Prüflicht durch die Mantelfläche (22) hindurch mindestens teilweise in das stabförmige Produkt (11) eindringt und mindestens teilweise durch eine Stirnseite (23, 24) wieder austritt, insbesondere mit Prüflicht, dessen Strahlen unter einem Winkel von mindestens 30°, bevorzugt von mindestens 60°, besonders bevorzugt von 90° zur Längserstreckung des stabförmigen Produkts (11) auf die Mantelfläche (22) auftreffen,
b) Erfassen des aus der Stirnseite (23, 24) des zu prüfenden stabförmigen Produkts (11) austretenden Prüflichts mit einem der Stirnseite (23, 24) zugeordneten, elektrooptischen Empfänger für das Prüflicht, dessen Hauptblickrichtung auf die Stirnseite (23, 24) gerichtet ist, insbesondere einer Kamera,
c) Auswerten des aus der Stirnseite (23, 24) ausgetretenen, von dem elektrooptischen Empfänger erfassten Prüflichts, insbesondere im Hinblick auf dessen Helligkeit bzw. Lichtintensität,
**dadurch gekennzeichnet, dass** eine Produktgruppe (10) aus stabförmigen Produkten (11) geprüft wird, die mehrere Produktlagen (18) von jeweils in einer Reihe nebeneinander angeordneten stabförmigen Produkten (11) aufweist, deren Stirnseiten (23, 24) an ihren gegenüberliegenden Enden jeweils eine von zwei gegenüberliegenden Produktgruppenseiten bilden.

2. Verfahren gemäß Anspruch 1, **dadurch gekennzeichnet, dass** die Helligkeit bzw. Lichtintensität des erfassten Prüflichts verglichen wird mit einem in einem Speicher hinterlegten Referenzwert oder mit der Helligkeit bzw. Lichtintensität von aus der anderen Stirnseite (23, 24) am gegenüberliegenden Ende desselben stabförmigen Produkts (11) austretendem Prüflicht oder von aus einer Stirnseite (23, 24) eines anderen stabförmigen Produkts (11) austretendem Prüflicht, dessen Mantelfläche (22) derart mit Prüflicht bestrahlt wird, dass das Prüflicht durch die Mantelfläche (22) hindurch mindestens teilweise in das stabförmige Produkt (11) eindringt und mindestens teilweise durch die Stirnseite (23, 24) wieder austritt, und dessen aus der Stirnseite (23, 24) austretendes Prüflicht mit einem oder dem elektrooptischen Empfänger erfasst und ausgewertet wird.

3. Verfahren gemäß einem der vorhergehenden Ansprüche, **dadurch gekennzeichnet, dass** die Mantelfläche (22) jedes stabförmigen Produkts (11) einer Produktlage (18) der Produktgruppe (10), vorzugsweise einer oder beider äußeren Produktlagen (18), mit Prüflicht bestrahlt wird, und das Prüflicht, das aus den Stirnseiten (23, 24) der stabförmigen Produkte (11) von mindestens einer der beiden gegenüberliegenden Produktgruppenseiten austritt, von einem bzw. dem diesen Stirnseiten (23, 24) zugeordneten elektrooptischen Empfänger empfangen und ausgewertet wird.

4. Verfahren gemäß einem der vorhergehenden Ansprüche, **dadurch gekennzeichnet, dass** jeder der gegenüberliegenden Stirnseiten (23, 24) der stabförmigen Produkte (11) jeweils ein insbesondere gesonderter elektrooptischer Empfänger zum Erfassen des jeweils austretenden Prüflichts zugeordnet ist, und dass sowohl das aus der einen als auch das aus der anderen der gegenüberliegenden Stirnseiten (23, 24) ausgetretene, von dem jeweils zugeordneten elektrooptischen Empfänger erfasste Prüflicht ausgewertet wird, und/oder dass die Hauptblickrichtung des oder jedes zugeordneten elektrooptischen Empfängers senkrecht zu den Stirnseite(n) (23, 24) der stabförmigen Produkte (11) verläuft, und/oder dass der oder der jeweilige zugeordnete elektrooptische Empfänger als Kamera ausgebildet ist, insbesondere als CCD- oder CMOS-Kamera, dass die oder die jeweilige Kamera ein insbesondere gemeinsames Ist-Bild der einen oder mehreren Stirnseite(n) (23, 24) aufnimmt, und dass das oder das jeweilige Ist-Bild ausgewertet wird.

5. Verfahren gemäß Anspruch 4, **dadurch gekennzeichnet, dass** im Rahmen der Auswertung des Ist-Bildes die Helligkeit oder Lichtintensität einer darin abgebildeten Stirnseite (23, 24) eines stabförmigen Produkts (11) verglichen wird mit einem hinterlegten Referenzwert, oder dass im Rahmen der Auswertung des Ist-Bildes die Helligkeit oder Lichtintensität einer darin abgebildeten Stirnseite (23, 24) verglichen wird mit der jeweiligen Helligkeit bzw. Lichtintensität mindestens einer anderen darin abgebildeten Stirnseite (23, 24) eines stabförmigen Produkts (11) derselben Produktlage (18) oder einer anderen Produktlage (18).

6. Verfahren gemäß Anspruch 4 oder 5, **dadurch gekennzeichnet, dass** jeder der Stirnseiten (23, 24) an den beiden Enden des stabförmigen Produkts (11) jeweils ein insbesondere gesonderter elektrooptischer, als Kamera ausgebildeter Empfänger zum Erfassen des jeweils austretenden Prüflichts zugeordnet ist, insbesondere derart, dass die Hauptblickrichtung der jeweiligen Kamera senkrecht zu der jeweils zugeordneten Stirnseite (23, 24) ausgerichtet ist, dass jede Kamera jeweils ein Ist-Bild derjenigen Stirnseite (23, 24) aufnimmt, der sie zugeordnet ist, und dass diese Ist-Bilder ausgewertet werden, indem die Helligkeit oder Lichtintensität der in dem einen Ist-Bild abgebildeten Stirnseite (23, 24) des einen Endes des stabförmigen Produkts (11) verglichen wird mit der Helligkeit bzw. Lichtintensität der in dem anderen Ist-Bild abgebildeten Stirnseite (23, 24) des anderen Endes des stabförmigen Produkts (11).

7. Verfahren gemäß Anspruch 5 oder 6, **dadurch gekennzeichnet, dass** ein Fehlersignal erzeugt wird für den Fall, dass im Rahmen des Vergleichs der Helligkeit der einen Stirnseite (23, 24) mit der jeweiligen Helligkeit bzw. Lichtintensität der anderen Stirnseite (23, 24) ein Unterschied zwischen den Helligkeits- bzw. Lichtintensitätswerten festgestellt wird, der über ein vorbestimmtes Maß hinaus geht.

8. Verfahren gemäß einem der vorhergehenden Ansprüche, **dadurch gekennzeichnet, dass** die Mantelfläche (22) des jeweiligen stabförmigen Produkts (11) aus zwei verschiedenen Richtungen mit Prüflicht beaufschlagt wird, insbesondere aus um 180° entgegengesetzten Richtungen, oder dass die Mantelflächen (22) der stabförmigen Produkte (11) zweier äußerer Produktlagen (18) der Produktgruppe (10) jeweils von unterschiedlichen Seiten aus mit Prüflicht beaufschlagt werden, wobei das Prüflicht, mit dem die eine äußere Produktlage (18) bestrahlt wird, aus einer anderen Richtung stammt als das Prüflicht, mit dem die andere äußere Produktlage (18) bestrahlt wird, insbesondere aus um 180° entgegengesetzten Richtungen.

9. Verfahren gemäß einem der vorhergehenden Ansprüche, **dadurch gekennzeichnet, dass** für den Fall, dass ein oder mehrere stabförmige Produkte (11) mit jeweils einem insbesondere außermittig angeordneten Abschnitt von rauchbarem Material geprüft werden, die jeweilige Mantelfläche (22) des jeweiligen stabförmigen Produkts (11) derart mit Prüfstrahlen des Prüflichts bestrahlt wird, dass die Prüfstrahlen nach dem Eindringen in die Mantelfläche (22) auf ihrem Weg zu dem elektrooptischen Empfänger in einer ersten Orientierung des jeweiligen stabförmigen Produkts (11) kein rauchbares Material oder einen kürzeren Abschnitt rauchbaren Materials innerhalb des stabförmigen Produkts (11) durchdringen oder durchdringen würden, während sie in einer zweiten, um 180° entgegengesetzten Orientierung des jeweiligen stabförmigen Produkts (11) rauchbares Material bzw. einen längeren Abschnitt rauchbaren Materials innerhalb des stabförmigen Produkts (11) durchdringen oder durchdringen würden.

10. Vorrichtung zur Prüfung von stabförmigen Produkten (11) der Zigarettenindustrie, insbesondere von Tabaksticks, mit jeweils einer insbesondere zylindrischen Mantelfläche (22) und zwei an gegenüberliegenden Enden angeordneten, insbesondere durch zwei Filterelemente vorzugsweise unterschiedlicher Länge gebildeten, insbesondere visuell identischen Stirnseiten (23, 24), wobei das stabförmige Produkt (11) zwischen den beiden Stirnseiten (23, 24), insbesondere in einem außermittig angeordneten Abschnitt, rauchbares Material aufweist, insbesondere Tabak, bevorzugt zur Durchführung des Verfahrens gemäß einem oder mehreren der vorhergehenden Ansprüche, mit mindestens einer Beleuchtungseinrichtung (17), von der Prüflicht ausgeht, das auf die Mantelfläche (22) mindestens eines zu prüfenden stabförmigen Produkts (11) gerichtet ist, bevorzugt derart, dass dessen Strahlen unter einem Winkel von mindestens 30°, bevorzugt von mindestens 60°, besonders bevorzugt von 90° zur Längserstreckung des stabförmigen Produkts (11) auf die Mantelfläche (22) auftreffen, wobei das Prüflicht durch die Mantelfläche (22) hindurch mindestens teilweise in das stabförmige Produkt (11) eindringt und mindestens teilweise durch die Stirnseite (23, 24) wieder austritt, mit mindestens einem elektrooptischen Empfänger, der derart angeordnet ist, dass dessen Hauptblickrichtung zur Erfassung desselben auf die Stirnseite (23, 24) des stabförmigen Produkts (11) gerichtet ist, sodass das aus der Stirnseite (23, 24) des stabförmigen Produkts (11) austretende Prüflicht erfasst wird, und mit einer Auswerteeinrichtung, mit der das von dem elektrooptischen Empfänger empfangene Prüflicht auswertbar ist, insbesondere im Hinblick auf dessen Helligkeit bzw. Lichtintensität, **dadurch gekennzeichnet, dass** das Prüflicht auf eine Produktgruppe (10) aus stabförmigen Produkten (11) gerichtet ist, die mehrere Produktlagen (18) von jeweils in einer Reihe nebeneinander angeordneten stabförmigen Produkten (11) aufweist, deren Stirnseiten (23, 24) an ihren gegenüberliegenden Enden jeweils eine von zwei gegenüberliegenden Produktgruppenseiten bilden.

11. Vorrichtung gemäß Anspruch 10, **dadurch gekennzeichnet, dass** die Beleuchtungseinrichtung (17) derart ausgebildet ist, dass die Mantelflächen (22) der stabförmigen Produkte (11) mindestens einer Produktlage (18), insbesondere einer oder beiden äußeren Produktlagen (18), mit dem Prüflicht beaufschlagbar sind, während sich die Produktgruppe (10) in einer Aufnahme eines Förderers (12) befindet, insbesondere in der Tasche eines drehbaren Revolvers.

12. Vorrichtung gemäß Anspruch 11, **dadurch gekennzeichnet, dass** die Beleuchtungseinrichtung (17) die Mantelflächen (22) der stabförmigen Produkte (11) einer ersten äußeren Produktlage (18) der Produktgruppe (11) mit Prüflicht beaufschlagt und die Mantelflächen (22) der stabförmigen Produkte (11) einer zweiten äußeren Produktlage (18) der Produktgruppe (10), indem die Beleuchtungseinrichtung (17) eine Lichtleiteinrichtung aufweist mit ein oder mehreren Lichtleitorganen (38), wie etwa Spiegeln oder Lichtwellenleitern, die von einer Lichtquelle (31) der Beleuchtungseinrichtung (17) ausgehendes Prüflicht auf die Mantelflächen (22) der ersten äußeren Produktlage (18) der Produktgruppe (10) leitet und auf die Mantelflächen (22) der zweiten äußeren Produktlage (18) der Produktgruppe (10), oder die von einer ersten Lichtquelle (31) der Beleuchtungseinrichtung (17) ausgehendes Prüflicht auf die Mantelflächen (22) der ersten äußeren Produktlage (18) leitet und von einer zweiten Lichtquelle (31) der Beleuchtungseinrichtung (17) ausgehendes Prüflicht zu den Mantelflächen (22) der zweiten äußeren Produktlage (18).

13. Vorrichtung gemäß Anspruch 12, **dadurch gekennzeichnet, dass** die Lichtleitorgane (38) der Lichtleiteinrichtung der Beleuchtungseinrichtung (17) benachbart zu unterschiedlichen Seiten der Produktgruppe (10) angeordnet sind, insbesondere derart, das Prüflicht aus unterschiedlichen, bevorzugt aus um 180° entgegengesetzten Richtungen die Mantelflächen (22) der ersten bzw. der zweiten Produktlage (18) beaufschlagt.

14. Vorrichtung gemäß Anspruch 11, **dadurch gekennzeichnet, dass** die Beleuchtungseinrichtung (17) die Mantelflächen (22) der stabförmigen Produkte (11) einer ersten äußeren Produktlage (18) der Produktgruppe (10) mit Prüflicht beaufschlagt und die Mantelflächen (22) der stabförmigen Produkte (11) einer zweiten äußeren Produktlage (18) der Produktgruppe (10), indem die Beleuchtungseinrichtung (17) zwei Lichtquellen (31) aufweist, die benachbart zu unterschiedlichen Seiten der Produktgruppe (10) angeordnet sind, sodass von der ersten Lichtquelle (31) der Beleuchtungseinrichtung (17) ausgehendes Prüflicht direkt auf die Mantelflächen (22) der ersten äußeren Produktlage (18) fällt und von der zweiten Lichtquelle (31) der Beleuchtungseinrichtung (17) ausgehendes Prüflicht direkt auf die Mantelflächen (22) der zweiten äußeren Produktlage (18), insbesondere derart, dass Prüflicht aus unterschiedlichen, bevorzugt aus um 180° entgegengesetzten Richtungen die Mantelflächen (22) der ersten bzw. der zweiten Produktlage (18) beaufschlagt.

15. Vorrichtung gemäß einem der vorhergehenden Ansprüche 10-14, **dadurch gekennzeichnet, dass** jeder der gegenüberliegenden Stirnseiten (23, 24) der stabförmigen Produkte (11) jeweils ein insbesondere gesonderter elektrooptischer Empfänger zum Erfassen des jeweils austretenden Prüflichts zugeordnet ist, insbesondere derart, dass die Hauptblickrichtung des jeweiligen elektrooptischen Empfängers senkrecht zu der den jeweils zugeordneten Stirnseiten (23, 24) ausgerichtet ist und/oder dass jeder Produktgruppenseite, die von den Stirnseiten (23, 24) der gegenüberliegenden Enden der stabförmigen Produkte (11) gebildet wird, ein gesonderter elektrooptischer Empfänger zum Erfassen des jeweils aus sämtlichen Stirnseiten (23, 24) der jeweiligen Produktgruppenseite austretenden Prüflichts zugeordnet ist, insbesondere derart, dass die Hauptblickrichtung des jeweiligen elektrooptischen Empfängers senkrecht zu den Stirnseiten (23, 24) der jeweiligen Produktgruppenseite ausgerichtet ist.

## Claims

1. A method for examining rod-shaped products (11) of the cigarette industry, in particular tobacco sticks, each having an in particular cylindrical lateral surface (22) and two end faces (23, 24), which are arranged at opposite ends, are formed in particular by two filter elements, preferably of different lengths, and are in particular visually identical, wherein the rod-shaped product (11) has smokable material, in particular tobacco, between the two end faces (23, 24), preferably in a section arranged off-centre, having the following measures:
a) irradiating the lateral surface (22) of at least one rod-shaped product (11) to be examined using examination light in such a way that the examination light penetrates through the lateral surface (22) at least partially into the rod-shaped product (11) and at least partially exits again through one end face (23, 24), in particular using examination light, the beams of which are incident on the lateral surface (22) at an angle of at least 30°, preferably of at least 60°, particularly preferably 90° in relation to the longitudinal extension of the rod-shaped product (11),
b) acquiring the examination light exiting from the end face (23, 24) of the rod-shaped product (11) to be examined using an electro-optical receiver for the examination light assigned to the end face (23, 24), the main viewing direction of which is directed onto the end face (23, 24), in particular a camera,
c) evaluating the examination light exiting from the end face (23, 24) and acquired by the electro-optical receiver, in particular with regard to its brightness or light intensity,
**characterized in that** a product group (10) made of rod-shaped products (11) is examined, which has a plurality of product layers (18) of rod-shaped products (11) each arranged adjacent to one another in a row, the end faces (23, 24) of which each form one of two opposite product group sides at their opposite ends.

2. The method as claimed in claim 1, **characterized in that** the brightness or light intensity of the acquired examination light is compared to a reference value stored in a memory or to the brightness or light intensity of examination light exiting from the other end face (23, 24) at the opposite end of the same rod-shaped product (11) or of examination light exiting from an end face (23, 24) of another rod-shaped product (11), the lateral surface (22) of which is irradiated using examination light in such a way that the examination light penetrates through the lateral surface (22) at least partially into the rod-shaped product (11) and at least partially exits again through the end face (23, 24), and the examination light of which exiting from the end face (23, 24) is acquired and evaluated using a or the electro-optical receiver.

3. The method as claimed in one of the preceding claims, **characterized in that** lateral surface (22) of each rod-shaped product (11) of a product layer (18) of the product group (10), preferably one or both outer product layers (18), is irradiated using examination light, and the examination light which exits from the end faces (23, 24) of the rod-shaped products (11) from at least one of the two opposite product group sides is received and evaluated by a or the electro-optical receiver assigned to these end faces (23, 24).

4. The method as claimed in one of the preceding claims, **characterized in that** each of the opposite end faces (23, 24) of the rod-shaped products (11) is respectively assigned an in particular separate electro-optical receiver for acquiring the respective exiting examination light, and that the examination light exiting from the one and also from the other of the opposite end faces (23, 24) and acquired by the respective assigned electro-optical receiver is evaluated, and/or **in that** the main viewing direction of the or each assigned electro-optical receiver extends perpendicularly to the end face(s) (23, 24) of the rod-shaped products (11), and/or **in that** the or the respective assigned electro-optical receiver is designed as a camera, in particular as a CCD or CMOS camera, that the or the respective camera records an in particular common actual image of the one or more end face(s) (23, 24), and that the or the respective actual image is evaluated.

5. The method as claimed in claim 4, **characterized in that,** in the scope of the evaluation of the actual image, the brightness or light intensity of an end face (23, 24) of a rod-shaped product (1) depicted therein is compared to a stored reference value, or that in the scope of the evaluation of the actual image, the brightness or light intensity of an end face (23, 24) depicted therein is compared to the respective brightness or light intensity of at least one other end face (23, 24) of a rod-shaped product (11) depicted therein of the same product layer (18) or another product layer (18).

6. The method as claimed in claim 4 or 5, **characterized in that** each of the end faces (23, 24) at the two ends of the rod-shaped products (11) is respectively assigned an in particular separate electro-optical receiver, designed as a camera, for acquiring the respective exiting examination light, in particular in such a way that the main viewing direction of the respective camera is oriented perpendicularly to the respective assigned end face (23, 24), that each camera respectively records an actual image of that end face (23, 24) to which it is assigned, and that these actual images are evaluated **in that** the brightness or light intensity of the end face (23, 24) of the one end of the rod-shaped product (11) depicted in the one actual image is compared to the brightness or light intensity of the end face (23, 24) of the other end of the rod-shaped product (11) depicted in the other actual image.

7. The method as claimed in claim 5 or 6, **characterized in that** an error signal is generated for the case that in the context of the comparison of the brightness of the one end face (23, 24) to the respective brightness or light intensity of the other end face (23, 24), a difference between the brightness or light intensity values is established which goes beyond a predetermined amount.

8. The method as claimed in one of the preceding claims, **characterized in that** examination light is applied to the lateral surface (22) of the respective rod-shaped product (11) from two different directions, in particular from directions opposite by 180°, or that examination light is applied from different sides to each of the lateral surfaces (22) of the rod-shaped products (11) of two outer product layers (18) of the product group (10), wherein the examination light, using which the one outer product layer (18) is irradiated, originates from a different direction than the examination light using which the other outer product layer (18) is irradiated, in particular from directions opposite by 180°.

9. The method as claimed in one of the preceding claims, **characterized in that** for the case that one or more rod-shaped products (11) each having a section of smokable material, which is in particular arranged off-centre, are examined, the respective lateral surface (22) of the respective rod-shaped product (11) is irradiated using examination beams of the examination light in such a way that the examination beams, after the penetration into the lateral surface (22), on their way to the electro-optical receiver in a first orientation of the respective rod-shaped product (11), do not penetrate or would not penetrate any smokable material or a shorter section of smokable material inside the rod-shaped product (11), while in a second orientation of the respective rod-shaped product (11) opposite by 180°, they penetrate or would penetrate smokable material or a longer section of smokable material inside the rod-shaped product (11).

10. A device for examining rod-shaped products (11) of the cigarette industry, in particular of tobacco sticks, each having an in particular cylindrical lateral surface (22) and two end faces (23, 24), which are arranged at opposite ends, are formed in particular by two filter elements, preferably of different lengths, and are in particular visually identical, wherein the rod-shaped product (11) has smokable material, in particular tobacco, between the two end faces (23, 24), in particular in a section arranged off-centre, preferably for carrying out the method as claimed in one or more of the preceding claims, having at least one illumination unit (17), from which examination light originates, which is directed onto the lateral surface (22) of at least one rod-shaped product (11) to be examined, preferably in such a way that its beams are incident on the lateral surface (22) at an angle of at least 30°, preferably of at least 60°, particularly preferably 90° in relation to the longitudinal extension of the rod-shaped product (11), wherein the examination light penetrates through the lateral surface (22) at least partially into the rod-shaped product (11) and exits again at least partially through the end face (23, 24), having at least one electro-optical receiver, which is arranged in such a way that its main viewing direction is directed for the acquisition of this examination light on the end face (23, 24) of the rod-shaped product (11), so that the examination light exiting from the end face (23, 24) of the rod-shaped product (11) is acquired, and having an evaluation unit, using which the examination light received by the electro-optical receiver can be evaluated, in particular with regard to its brightness or light intensity, **characterized in that** the examination light is directed onto a product group (10) made up of rod-shaped products (11), which has a plurality of product layers (18) of rod-shaped products (11), each arranged in a row adjacent to one another, the end faces (23, 24) of which at their opposite ends each form one of two opposite product group sides.

11. The device as claimed in claim 10, **characterized in that** the illumination unit (17) is designed in such a way that the examination light can be applied to the lateral surfaces (22) of the rod-shaped products (11) of at least one product layer (18), in particular one or both outer product layers (18), while the product group (10) is located in a receptacle of a conveyor (12), in particular in the pocket of a rotatable revolver.

12. The device as claimed in claim 11, **characterized in that** the illumination unit (17) applies examination light to the lateral surfaces (22) of the rod-shaped products (11) of a first outer product layer (18) of the product group (11) and to the lateral surfaces (22) of the rod-shaped products (11) of a second outer product layer (18) of the product group (10), **in that** the illumination unit (17) has a light guiding unit having one or more light guiding elements (38), such as mirrors or optical fibres, which conducts examination light originating from a light source (31) of the illumination unit (17) onto the lateral surfaces (22) of the first outer product layer (18) of the product group (10) and onto the lateral surfaces (22) of the second outer product layer (18) of the product group (10), or conducts the examination light originating from a first light source (31) of the illumination unit (17) onto the lateral surfaces (22) of the first outer product layer (18) and examination light originating from a second light source (31) of the illumination unit (17) to the lateral surfaces (22) of the second outer product layer (18).

13. The device as claimed in claim 12, **characterized in that** the light guiding elements (38) of the light guiding unit of the illumination unit (17) are arranged adjacent to different sides of the product group (10), in particular in such a way that examination light is applied to the lateral surfaces (22) of the first or the second product layer (18), respectively, from different directions, preferably opposite by 180°.

14. The device as claimed in claim 11, **characterized in that** the illumination unit (17) applies examination light to the lateral surfaces (22) of the rod-shaped products (11) of a first outer product layer (18) of the product group (10) and to the lateral surfaces (22) of the rod-shaped products (11) of a second outer product layer (18) of the product group (10), **in that** the illumination unit (17) has two light sources (31), which are arranged adjacent to different sides of the product group (10), so that examination light originating from the first light source (31) of the illumination unit (17) is incident directly on the lateral surfaces (22) of the first outer product layer (18) and examination light originating from the second light source (31) of the illumination unit (17) is applied directly to the lateral surfaces (22) of the second outer product layer (18), in particular in such a way that examination light is applied to the lateral surfaces (22) of the first or the second product layer (18), respectively, from different directions, preferably opposite by 180°.

15. The device as claimed in one of the preceding claims 10-14, **characterized in that** each of the opposite end faces (23, 24) of the rod-shaped products (11) is respectively assigned an electro-optical receiver, which is separate in particular, for acquiring the respective exiting examination light, in particular in such a way that the main viewing direction of the respective electro-optical receiver is oriented perpendicularly to the respective assigned end faces (23, 24), and/or that each product group side which is formed by the end faces (23, 24) of the opposite ends of the rod-shaped products (11) is assigned a separate electro-optical receiver for acquiring the respective examination light exiting from all end faces (23, 24) of the respective product group side, in particular in such a way that the main viewing direction of the respective electro-optical receiver is oriented perpendicularly to the end faces (23, 24) of the respective product group side.

## Revendications

1. Procédé d'examen de produits en forme de tige (11) de l'industrie des cigarettes, en particulier de bâtonnets de tabac, qui comprennent respectivement une surface latérale en particulier cylindrique (22), et deux faces d'extrémité (23, 24) disposées à des extrémités opposées, formées en particulier par deux éléments filtrants de préférence de longueurs différentes, en particulier visuellement identiques, le produit en forme de tige (11) comportant une substance à fumer, en particulier du tabac, entre les deux faces d'extrémité (23, 24), de préférence dans une partie située de manière excentrée, comprenant les mesures suivantes :
a) irradiation de la surface latérale (22) d'au moins un produit en forme de tige (11) à examiner à l'aide d'une lumière d'examen, de telle sorte que la lumière d'examen pénètre au moins en partie dans le produit en forme de tige (11) à travers la surface latérale (22) et ressorte au moins en partie par une face d'extrémité (23, 24), en particulier à l'aide d'une lumière d'examen, dont les faisceaux sont incidents sur la surface latérale (22) sous un angle d'au moins 30°, de préférence d'au moins 60°, de manière particulièrement préférée de 90°, par rapport à l'étendue longitudinale du produit en forme de tige (11),
b) détection de la lumière d'examen sortant de la face d'extrémité (23, 24) du produit en forme de tige (11) à examiner à l'aide d'un récepteur électro-optique destiné à la lumière d'examen et associé à la face d'extrémité (23, 24), dont la direction d'observation principale est dirigée vers la face d'extrémité (23, 24), en particulier d'une caméra,
c) évaluation de la lumière d'examen sortant de la face d'extrémité (23, 24) et détectée par le récepteur électro-optique, en particulier en ce qui concerne sa luminosité ou son intensité lumineuse,
**caractérisé en ce qu'**un groupe de produits (10) constitué de produits en forme de tige (11) est examiné, lequel présente plusieurs couches de produits (18) respectivement constituées de produits en forme de tige (11) disposés les uns à côté des autres en une rangée, dont les faces d'extrémité (23, 24) forment respectivement, à leurs extrémités opposées, l'une de deux faces opposées du groupe de produits.

2. Procédé selon la revendication 1, **caractérisé en ce que** la luminosité ou l'intensité lumineuse de la lumière d'examen détectée est comparée à une valeur de référence mémorisée dans une mémoire ou à la luminosité ou à l'intensité lumineuse de la lumière d'examen sortant de l'autre face d'extrémité (23, 24) à l'extrémité opposée du même produit en forme de tige (11) ou de la lumière d'examen sortant d'un face d'extrémité (23, 24) d'un autre produit en forme de tige (11) dont la surface latérale (22) est exposée à une lumière d'examen de telle sorte que la lumière d'examen pénètre au moins en partie dans le produit en forme de tige (11) à travers la surface latérale (22) et ressorte au moins en partie par la face d'extrémité (23, 24), et dont la lumière d'examen sortant de la face d'extrémité (23, 24) est détectée et évaluée à l'aide d'un ou du récepteur électro-optique.

3. Procédé selon l'une quelconque des revendications précédentes, **caractérisé en ce que** la surface latérale (22) de chaque produit en forme de tige (11) d'une couche de produits (18) du groupe de produits (10), de préférence de l'une ou des deux couches de produits (18) extérieures, est exposée à une lumière d'examen, et **en ce que** la lumière d'examen sortant des faces d'extrémité (23, 24) des produits en forme de tige (11) d'au moins l'une des deux faces opposées du groupe de produits est reçue et évaluée par un ou le récepteur électro-optique associé à ces faces d'extrémité (23, 24).

4. Procédé selon l'une quelconque des revendications précédentes, **caractérisé en ce qu'**à chacune des faces d'extrémité opposées (23, 24) des produits en forme de tige (11) est associé un récepteur électro-optique, en particulier séparé, pour détecter la lumière d'examen sortante respective, et **en ce que** la lumière d'examen, détectée par le récepteur électro-optique associé respectif, sortant de l'une ainsi que de l'autre des faces d'extrémité opposées (23, 24), est évaluée et/ou **en ce que** la direction d'observation principale du ou de chaque récepteur électro-optique associé s'étend perpendiculairement à la ou aux face(s) d'extrémité (23, 24) des produits en forme de tige (11), et/ou **en ce que** le ou les récepteurs électro-optiques respectivement associés est/sont réalisé(s) sous la forme d'une caméra, en particulier d'une caméra CCD ou CMOS, **en ce que** la ou les caméras respectives acquièrent une image réelle, en particulier commune, de la ou des face(s) d'extrémité (23, 24) et **en ce que** la ou les image(s) réelle(s) respective(s) est/sont évaluée(s).

5. Procédé selon la revendication 4, **caractérisé en ce que**, dans le cadre de l'évaluation de l'image réelle, la luminosité ou l'intensité lumineuse d'une face d'extrémité (23, 24) d'un produit en forme de tige (11) représenté dans celle-ci est comparée à une valeur de référence mémorisée, ou **en ce que**, dans le cadre de l'évaluation de l'image réelle, la luminosité ou l'intensité lumineuse d'une face d'extrémité (23, 24) représentée dans celle-ci est comparée à la luminosité ou à l'intensité lumineuse respective d'au moins une autre face d'extrémité (23, 24) représentée dans celle-ci d'un produit en forme de tige (11) de la même couche de produits (18) ou d'une autre couche de produits (18).

6. Procédé selon la revendication 4 ou 5, **caractérisé en ce qu'**à chacune des faces d'extrémité (23, 24), aux deux extrémités du produit en forme de tige (11), est respectivement associé un récepteur électro-optique, en particulier séparé, réalisé sous la forme d'une caméra, pour détecter la lumière d'examen sortante respective, en particulier de telle sorte que la direction d'observation principale de la caméra respective soit orientée perpendiculairement à la face d'extrémité (23, 24) associée respective, **en ce que** chaque caméra acquiert une image réelle de la face d'extrémité (23, 24) respective à laquelle elle est associée, et **en ce que** ces images réelles sont évaluées en comparant la luminosité ou l'intensité lumineuse de la face d'extrémité (23, 24), représentée dans ladite image réelle, de ladite extrémité du produit en forme de tige (11), à la luminosité ou à l'intensité lumineuse de la face d'extrémité (23, 24) de l'autre extrémité du produit en forme de tige (11), représentée dans l'autre image réelle.

7. Procédé selon la revendication 5 ou 6, **caractérisé en ce qu'**un signal d'erreur est généré dans le cas où, dans le cadre de la comparaison de la luminosité de l'une des faces d'extrémité (23, 24) à la luminosité ou à l'intensité lumineuse respective de l'autre face d'extrémité (23, 24), une différence entre les valeurs de luminosité ou d'intensité lumineuse est constatée, laquelle dépasse une valeur prédéterminée.

8. Procédé selon l'une quelconque des revendications précédentes, **caractérisé en ce que** la surface latérale (22) du produit en forme de tige (11) respectif est exposée à une lumière d'examen provenant de deux directions différentes, en particulier de directions opposées de 180°, ou **en ce que** les surfaces latérales (22) des produits en forme de tige (11) de deux couches de produits (18) extérieures du groupe de produits (10) sont respectivement exposées à une lumière d'examen provenant de faces différentes, la lumière d'examen avec laquelle ladite couche de produits (18) extérieure est irradiée provenant d'une direction différente de celle de la lumière d'examen avec laquelle l'autre couche de produits (18) extérieure est irradiée, en particulier depuis des directions opposées de 180°.

9. Procédé selon l'une quelconque des revendications précédentes, **caractérisé en ce que**, dans le cas où un ou plusieurs produits en forme de tige (11) sont respectivement examinés avec une partie de substance à fumer disposée en particulier de manière excentrée, la surface latérale (22) respective du produit en forme de tige (11) respectif est irradiée avec des faisceaux d'examen de la lumière d'examen de telle sorte que les faisceaux d'examen, après avoir pénétré dans la surface latérale (22), sur leur chemin vers le récepteur électro-optique, ne traversent aucune substance à fumer ou traversent une partie plus courte de substance à fumer à l'intérieur du produit en forme de tige (11) dans une première orientation du produit en forme de tige (11) respectif, alors qu'ils traversent ou traverseraient la substance à fumer ou une partie plus longue de substance à fumer à l'intérieur du produit en forme de tige (11) dans une seconde orientation du produit en forme de tige (11) respectif, opposée de 180°.

10. Dispositif d'examen de produits en forme de tige (11) de l'industrie des cigarettes, en particulier de bâtonnets de tabac, présentant respectivement une surface latérale en particulier cylindrique (22) et deux faces d'extrémité (23, 24) disposées à des extrémités opposées, formées en particulier par deux éléments filtrants de préférence de longueurs différentes, en particulier visuellement identiques, le produit en forme de tige (11) comportant une substance à fumer, en particulier du tabac, entre les deux faces d'extrémité (23, 24), en particulier dans une partie située de manière excentrée, de préférence pour la mise en œuvre du procédé selon l'une ou plusieurs des revendications précédentes, comprenant au moins un dispositif d'éclairage (17) duquel sort une lumière d'examen qui est dirigée vers la surface latérale (22) d'au moins un produit en forme de tige (11) à examiner, de préférence de telle sorte que ses faisceaux soient incidents sur la surface latérale (22) sous un angle d'au moins 30°, de préférence d'au moins 60°, de manière particulièrement préférée de 90°, par rapport à l'étendue longitudinale du produit en forme de tige (11), la lumière d'examen pénétrant au moins en partie dans le produit en forme de tige (11) à travers la surface latérale (22) et ressortant au moins en partie par la face d'extrémité (23, 24), comprenant au moins un récepteur électro-optique disposé de telle sorte que sa direction d'observation principale soit dirigée vers le face d'extrémité (23, 24) du produit en forme de tige (11) pour détecter celui-ci, de telle sorte que la lumière d'examen sortant de la face d'extrémité (23, 24) du produit en forme de tige (11) soit détectée, et comprenant un dispositif d'évaluation avec lequel la lumière d'examen reçue par le récepteur électro-optique peut être évaluée, en particulier en ce qui concerne sa luminosité ou son intensité lumineuse, **caractérisé en ce que** la lumière d'examen est dirigée vers un groupe de produits (10) en forme de tige (11), lequel présente plusieurs couches de produits (18) respectivement constituées de produits en forme de tige (11) disposés les uns à côté des autres en une rangée, dont les faces d'extrémité (23, 24) forment respectivement, à leurs extrémités opposées, l'une de deux faces opposées du groupe de produits.

11. Dispositif selon la revendication 10, **caractérisé en ce que** le dispositif d'éclairage (17) est conçu de telle sorte que les surfaces latérales (22) des produits en forme de tige (11) d'au moins une couche de produits (18), en particulier d'une ou des deux couches de produits (18) extérieures, peuvent être exposées à la lumière d'examen, alors que le groupe de produits (10) se trouve dans un réceptacle d'un convoyeur (12), en particulier dans l'alvéole d'une tourelle rotative.

12. Dispositif selon la revendication 11, **caractérisé en ce que** le dispositif d'éclairage (17) expose à une lumière d'examen les surfaces latérales (22) des produits en forme de tige (11) d'une première couche de produits (18) extérieure du groupe de produits (11) et les surfaces latérales (22) des produits en forme de tige (11) d'une seconde couche de produits (18) extérieure du groupe de produits (10), **en ce que** le dispositif d'éclairage (17) comporte un dispositif de guidage de lumière comprenant un ou plusieurs organes de guidage de lumière (38), tels que des miroirs ou des guides d'ondes optiques, qui guident la lumière d'examen provenant d'une source lumineuse (31) du dispositif d'éclairage (17) vers les surfaces latérales (22) de la première couche de produits (18) extérieure du groupe de produits (10) et vers les surfaces latérales (22) de la seconde couche de produits (18) extérieure du groupe de produits (10), ou guide la lumière d'examen provenant d'une première source lumineuse (31) du dispositif d'éclairage (17) vers les surfaces latérales (22) de la première couche de produits (18) extérieure et la lumière d'examen provenant d'une seconde source lumineuse (31) du dispositif d'éclairage (17) vers les surfaces latérales (22) de la seconde couche de produits (18) extérieure.

13. Dispositif selon la revendication 12, **caractérisé en ce que** les organes de guidage de lumière (38) du dispositif de guidage de lumière du dispositif d'éclairage (17) sont disposés à proximité de différentes faces du groupe de produits (10), en particulier de telle sorte que la lumière d'examen soit incidente sur les surfaces latérales (22) de la première ou de la seconde couche de produits (18) depuis des directions différentes, de préférence depuis des directions opposées de 180°.

14. Dispositif selon la revendication 11, **caractérisé en ce que** le dispositif d'éclairage (17) expose à une lumière d'examen les surfaces latérales (22) des produits en forme de tige (11) d'une première couche de produits (18) extérieure du groupe de produits (10) et les surfaces latérales (22) des produits en forme de tige (11) d'une seconde couche de produits (18) extérieure du groupe de produits (10), **en ce que** le dispositif d'éclairage (17) comporte deux sources lumineuses (31) disposées à proximité de différentes faces du groupe de produits (10), de telle sorte que la lumière d'examen provenant de la première source lumineuse (31) du dispositif d'éclairage (17) soit incidente directement sur les surfaces latérales (22) de la première couche de produits (18) extérieure et que la lumière d'examen provenant de la seconde source lumineuse (31) du dispositif d'éclairage (17) soit incidente directement sur les surfaces latérales (22) de la seconde couche de produits (18) extérieure, en particulier de telle sorte que la lumière d'examen soit incidente sur les surfaces latérales (22) de la première ou de la seconde couche de produits (18) depuis des directions différentes, de préférence depuis des directions opposées de 180°.

15. Dispositif selon l'une quelconque des revendications précédentes 10 à 14, **caractérisé en ce qu'**un récepteur électro-optique, en particulier séparé, est associé à chacune des faces d'extrémité (23, 24) opposées des produits en forme de tige (11) pour détecter la lumière d'examen sortante, en particulier de telle sorte que la direction d'observation principale du récepteur électro-optique respectif soit perpendiculaire aux faces d'extrémité (23, 24) associées et/ou **en ce qu'**à chaque face de groupe de produits, formée par les faces d'extrémité (23, 24) des extrémités opposées des produits en forme de tige (11), est associé un récepteur électro-optique séparé pour détecter la lumière d'examen sortant de toutes les faces d'extrémité (23, 24) de la face respective du groupe de produits, en particulier de telle sorte que la direction d'observation principale du récepteur électro-optique respectif soit orientée perpendiculairement aux faces d'extrémité (23, 24) de la face respective du groupe de produits.
